# FASCICULE DE BREVET EUROPEEN

(11) **EP 1 530 480 B1**
(45) Date de publication et mention de la délivrance du brevet: **30.09.2009**
(21) Numéro de dépôt: 03747478.0
(22) Date de dépôt: 29.04.2003
(51) Int. Cl.: A61K 8/97, A61K 8/73, A61K 31/732, A61K 36/888, A61Q 19/08, A61Q 19/00

(54) **COMPOSITION COSMETIQUE COMPRENANT DES MONO- OU POLY-SACCHARIDES, UTILISATIONS ET PROCEDES DE TRAITEMENT**
KOSMETISCHE ZUSAMMENSETZUNG MIT MONOSACCHARIDEN ODER POLYSACCHARIDEN, ANWENDUNGEN UND BEHANDLUNGSVERFAHREN
COSMETIC COMPOSITION COMPRISING MONOSACCHARIDES OR POLYSACCHARIDES, USES AND TREATMENT METHODS

(30) Priorité: 30.04.2002 FR 0205407; 30.04.2002 FR 0205408
(43) Date de publication de la demande: 18.05.2005
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2003/001348
(87) Numéro de publication internationale: WO 2003/092645

(56) Documents cités:
- DE-C- 4 133 920
- DATABASE CAPLUS [en ligne] CHEMICAL ABSTRACTS SERVICE, COLUMBUS, OHIO, US; 2000 retrieved from STN Database accession no. 2000:834255 XP002227093
- DATABASE CAPLUS [en ligne] CHEMICAL ABSTRACTS SERVICE, COLUMBUS, OHIO, US; 1974 retrieved from STN Database accession no. 1974:118284 XP002227094
- DATABASE CAPLUS [en ligne] CHEMICAL ABSTRACTS SERVICE, COLUMBUS, OHIO, US; 1990 retrieved from STN Database accession no. 1990:503209 XP002227095
- DATABASE WPI Week 200039 Derwent Publications Ltd., London, GB; AN 1998-589663 XP002227096 & JP 10 265399 A (AGENCY OF IND SCI & TECHNOLOGY), 6 octobre 1998 (1998-10-06)
- DATABASE WPI Week 199717 Derwent Publications Ltd., London, GB; AN 1997-180067 XP002227097 & CN 1 092 997 A (CHEN), 5 octobre 1994 (1994-10-05)
- PATENT ABSTRACTS OF JAPAN vol. 1997, no. 11 (C & JP 09 194334 A (NARISU KESHOHIN KK), 29 juillet 1997 (1997-07-29)
- DATABASE WPI Week 199145 Derwent Publications Ltd., London, GB; AN 1991-329195 XP002227098 & JP 03 220129 A (SHISEIDO CO), 27 septembre 1991 (1991-09-27)
- DATABASE WPI Section Ch, Week 200330 Derwent Publications Ltd., London, GB; Class B03, AN 2003-309963 XP002258404 & RU 2 190 666 C (AS RUSSIA URALS KOMI RES CENTRE PHYSIOL), 10 octobre 2002 (2002-10-10)
- DATABASE WPI Section Ch, Week 200064 Derwent Publications Ltd., London, GB; Class B04, AN 2000-662809 XP002258407 & RU 2 149 642 C1 (AS RUSSIA URALS KOMI RES CENTRE PHYSIOL), 27 mai 2000 (2000-05-27)

## Description

La présente invention concerne les domaines de la cosmétique et de la dermatologie, et a pour objet l'utilisation d' au moins un mono- ou poly-saccharide, constitutif d'une substance pectique provenant d'une plante de la famille des Lemnacées, comme ingrédient actif dans une composition cosmetique et/ou pharmaceutique.

Les plantes de la famille des Lemnacées sont des plantes angiospermes monocotylédones. La famille de Lemnacées appartient à l'ordre des Arales et possède quatre genres : Lemna, Spirodela, Wolffia et Wolffiela. On lui attribue, dans le monde, jusqu'à une trentaine d'espèces. Cinq d'entre elles font partie de la flore européenne : *Lemna triscula, Lemna minor, Lemna gibba, Spirodella polyrrhiza, Wolffia arrhiza*

Les plantes de la famille des Lemnacées sont communément appelées « lentilles d'eau » ou lenticule mineure. Ce sont de petites plantes aquatiques vivaces, flottant à la surface des eaux ou parfois entre deux eaux, sans tiges ni feuilles, réduites à un simple corpuscule lenticulaire horizontal et arrondi, appelé fronde. Elles possèdent une ou plusieurs racines filiformes, non ramifiées et sans poils absorbants, jouant un rôle d'ancrage.

Les lentilles d'eau ont diverses utilisations, elles sont parfois produites pour un usage alimentaire ; récoltées et séchées, elles sont vendues dans certain magasin diététique. Dans certains pays en voie de développement, les lentilles d'eau sont utilisées dans l'alimentation animale, comme par exemple pour nourrir les volailles ou les poissons. Leur succès est dû à leur teneur très élevée en protéine, les protéines représentent en effet 35 à 50 % du poids sec, soit autant que le taux de protéine présent dans les graines de soja. Par ailleurs, les lentilles d'eau ont d'autres avantages déterminants : par leur croissance très rapide, elles contribuent à dépolluer les eaux stagnantes ; elles sont ainsi utilisées efficacement en matière d'assainissement.

Les plantes de la famille des lemnacées (comme toutes les plantes) ont la particularité de posséder des polysaccharides particuliers composant la structure des parois cellulaires végétales : les pectines. Les pectines des lentilles d'eau sont particulières, notamment par leur composition. Elles sont appelées apiogalactoronan ou lemnan, en ce qui concerne les pectines des plantes de l'espèce *Lemna minor*.

Les substances pectiques, ou pectines, sont des macromolécules de nature glucidiques, composées essentiellement d'acides galacturoniques.

Le motif monomère des substances pectiques est l'acide αD-galaturonique, le squelette des substances pectiques est donc formé par des acides galactumniques liés en α [1-4], cette chaîne constituant l'acide polygalacturonique ou acide pectique. En général, les substances pectiques ont une structure plus complexe résultant de la substitution de certains groupes sur la chaîne principale.

Les lentilles d'eau possèdent une substance pectique particulière: l'apiogalactoronan. La substance pectique de la lentille d'eau *Lemna minor* est ramifiée. Cet apiogalactoronan a été isolé de la lentille d'eau et caractérisé pour la première fois par Hart et Kindel en 1970.

La composition de l'aplogalactoroinan est particulière. En effet, la chaîne saccharidique de la substance pectique de *Lemna minor* (ou lemnan) est composée d'acide D-galacturorlique (64 %) et de D-apiose (25 %) comme constituants principaux, ainsi que de galactose, d'arabinose, de rhamonose et de xylose.

L'apiose est un sucre peu commun, c'est un pentose à chaîne branchée. Il a été mis en évidence la première fois par *Bell et al*. (1954) comme un constituant des polysaccharides de *Posidionia australis.*

Il est connu d'utiliser des substances pectiques dans de nombreux domaines, notamment dans certains domaines thérapeutiques où ils sont utilisés en tant qu'agent épaississant ou comme excipaient (DE4133920) . L'utilisation de polysaccharides pectiniques de la famille des Lemnacées est également connu en tant qu'agent immunostimulateur, notamment pour stimuler la phagocytose (Dvodova, R.G. et al. Russian Journal of Bioorganic Chemistry, 2000, 26(10), 669-676 ; RU2149642).

Toutefois une utilisation des saccharides, constitutifs d'une substance pectique provenant d'une plante de la famille des Lemnacées, comme principe actif, dans le domaine cosmétique et/ou pharmaceutique n'est pas encore connue.

Or, les inventeurs de la présente invention ont constaté, de manière surprenante et inattendue, que les mono- ou poly-saccharides constitutifs d'une substance pectique, provenant d'une plante de la famille des Lemnacées, présentent des activités biologiques spécifiques, qui les rendent directement utilisables dans les préparations ou les compositions cosmétiques, dermatologiques et pharmaceutiques à usage topique externe.

Ainsi le principal objet de la présente invention consiste en une utilisation d'au moins un mono- ou poly-saccharide, constitutifs d'une substance pectique provenant d'une plante de la formule des Lemnacées, comme ingredient actif dans une composition cosmetique et/ou pharmaceutique.

Les inventeurs ont notamment constaté que ces saccharides présentent, des actions anti-inflammatoires, apaisantes, hydratantes, protectrices ou encore anti-âge prononcées dans le cadre de l'utilisation précitée. Par ailleurs, ces saccharides ont un effet anti-hyaluronidase, c'est-à-dire qu'ils inhibent l'activité de la hyaluronidase, enzyme qui hydrolyse l'acide hyaluronique.

Préférentiellement, e principe actif selon l'invention comprendra la pectine contenue dans les plantes de la famille des Lemnacées.

Selon un mode de réalisation particulier de l'invention, le poly-saccharide provenant de la famille des Lemnacées est une substance pectique comprenant au moins un motif apiose.

Selon un autre moyen de réalisation de l'invention, le principe actif selon l'invention comprendra le principe actif un mono-saccharide, l'apiose, constitutif d'une substance pectique provenant de la famille des Lemnacées.

Par ailleurs, selon encore un mode de réalisation de l'invention, les plantes de la famille des Lemnacées sont des plantes du genre Lemna. Bien entendu les substances pectiques peuvent être obtenues à partir d'au moins l'une quelconque des nombreuses variétés et espèces appartenant au genre Lemna.

Préférentiellement, les substances pectiques sont obtenues à partir d'au moins un végétal de l'espèce *Lemna minor*.

Selon l'invention, le terme "principe actif" peut se définir comme étant toute molécule ou tout ensemble de molécule susceptible d'apporter des modifications ou des modulations au fonctionnement d'un système biologique.

Les composés actifs selon l'invention, c'est-à-dire les mono- ou poly-saccharides constitutifs d'une substance pectique, sont obtenus à partir de la pectine. Les mono- ou poly-saccharides constitutifs d'une substance pectique désignent toutes substances pectiques ainsi que tous les saccharides ou chaînes saccharidiques qui les composent. Les mono- ou poly-saccharides constitutifs d'une substance pectique désignent ainsi les pectines qui ont été plus ou moins hydrolysées.

Une caractéristique essentielle de l'invention est que ces substances pectiques, dont sont tirés les mono- ou poly-saccharides, proviennent d'une plante de la famille des Lemnacées.

Ainsi, toute méthode d'extraction et/ou de purification connue de l'homme du métier peut être utilisée pour préparer les composés glucidiques selon l'invention.

Bien entendu, la quantité efficace de principe actif constituant la composition correspond à la quantité nécessaire afin d'obtenir le résultat désiré.

Selon un mode de réalisation avantageux de l'invention, le mono- ou poly-saccharide, constitutif d'une substance pectique provenant d'une plante de la famille des Lemnacées, est présent en une quantité représentant de 10⁻⁵ % à 20 % du poids total de la composition et, préférentiellement, en une quantité représentant de 10⁻⁴ % à 5 % du poids total de la composition.

Selon un mode de réalisation avantageux de l'invention, les composés actifs précités sont préalablement solubilisés ou dispersés dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, les composés actifs précités sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

The composition comprenantt au moins un principe actif selon l'invention pourron être une composition cosmétique ou dermatologique ou pharmaceutique. Préférentiellement selon l'invention, la composition est une composition cosmétique, car elle est destinée à améliorer l'aspect et les performances cutanées générales de l'individu qui en fait usage.

La composition comprenant au moins un principe actif selon l'invention est préférentiellement une composition cosmétique et/ou dermatologique adaptée à l'administration par voie topique cutanée comprenant un milieu cosmétiquement ou pharmaceutiquement acceptable.

Il est bien évident que l'invention s'adresse aux mammifères en général et plus particulièrement aux êtres humains.

Quelle que soit la forme de l'invention, la composition comprenant au moins un principe actif selon l'invention, peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées.

Préférentiellement, les compositions comprenant au moins un principe actif selon la présente invention, se présenteront sous une forme galénique adaptée à l'administration par voie topique cutanée, et couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions pourront notamment se présenter sous forme d'une solution aqueuse, hydralcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Pour l'injection, la composition compenant au moin un principe actif selon l'invention, peut se présenter sous forme de lotion aqueuse, huileuse ou sous forme de sérum. Pour l'application sur les yeux, la composition peut se présenter sous forme de gouttes et pour l'ingestion, elle peut se présenter sous forme de capsules, de granulés, de sirops ou de comprimés.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition.

Lorsque la composition comprenant au moins un principe actif de l'invention, est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions comprenant au moins un principe actif selon l'invention, trouvent une application notamment comme compositions cosmétiques ou pharmaceutiques pour la peau, les muqueuses et/ou les semi-muqueuses.

Elles trouvent une application toute particulière en tant que produit de protection et/ ou de soin de la peau, ou encore en tant que composition anti-ride et/ou anti-âge.

On peut également envisager une application dans le domaine des compositions de maquillage de la peau du visage et du corps, telles que les rouges à lèvres, les fonds de teint, les crèmes teintées, les sticks anti-cernes, ou les compositions anti-solaires ou de bronzage artificiel.

Les cornpositions, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps.

La composition comprenant au moins un principe actif selon l'invention, peut également consister en des préparations solides comprenant également des savons ou des pains de nettoyage.

La composition comprenant ou moins un principe actif selon l'invention, peut être aussi conditionnée sous forme d'une composition pour aérosol comprenant également un agent propulseur sous pression.

La compostion comprenant au moins un principe actif selon l'invention, peut être aussi à usage bucco-dentaire, par exemple une pâte de dentifrice. La composition peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule, ou encore d'un complément nutritionnel.

Selon l'invention, on peut, entre autres, ajouter à la composition d'autres agents actifs destinés notamment à la prévention et/ou au traitement des manifestations cutanées du vieillissement et/ou à la protection de la peau contre les agressions extérieures.

L'invention a également pour objet l'utilisation, comme ingrédient actif, d'au moins un mono- ou poly-saccharide, constitutif d'une substance pectique provenant d'une plante de la famille des Lemnacées, tel que défini précédemment, dans ou pour la préparation d'une composition, les composés ou la composition étant destinés à traiter, de manière curative et/ou préventive, les signes cutanés du vieillissement ainsi qu'à améliorer l'aspect de la peau et/ou des phanères.

D'une manière plus générale, la composition comprenant au moins un principe actif selon l'invention, selon l'invention aura une activité efficace dans tous les soins de la peau et/ou des phanères

Par les soins de la peau et/ou des phanères, on entend toutes les actions destinées à conserver ou à rétablir un bon fonctionnement de la peau et/ou des phanères ou encore tout moyen qui sert à préserver on à améliorer leur apparence et/ou leur aspect.

Ainsi le soin inclut l'hydratation, l'apaisement, la protection contre tous types d'agression, notamment la protection solaire, la lutte et la prévention des manifestations du vieillissement, notamment les manifestations cutanées du vieillissement.

Par manifestations cutanées du vieillissement on entend toutes modifications de l'aspect extérieur de la peau dues au vieillissement comme, par exemple, les rides et ridules, la peau flétrie, la peau molle, la peau amincie le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets.

Par l'expression "améliorer l'aspect de la peau", on entend tous les phénomènes qui sont susceptibles d'avoir pour conséquence une amélioration visuelle de l'état de la peau. La peau présentera une meilleure apparence ; elle sera, par exemple, beaucoup plus belle, ferme et/ou lisse. Toutes les petites imperfections seront diminuées ou supprimées. L'aspect papyracé de la peau sera, par exemple, atténué.

Par ailleurs, selon une caractéristique essentielle, l'invention concerne l'utilisation d'au moins un mono- ou poly-saccharide, constitutif d'une substance pectique provenant d'une plante de la famille des Lemnacées afin de maintenir et/ou de stimuler l'hydratation cutanée et/ou afin de lutter contre le dessèchement de la peau.

En effet, les mono- ou poly-saccharides, constitutifs d'une substance pectique provenant d'une plante de la famille des Lemnacées, ont une action anti-hyaluronidase. C'est-à-dire qu'ils inhibent l'activité des enzymes, les hyaluronidases, qui dégradent l'acide hyaluronique. Or, l'acide hyaluronique est le principal glycosaminoglycanne du derme et il exerce un rôle très important. Il est en effet connu que le vieillissement de l'épiderme, ainsi que le dessèchement observé en particulier dans les peaux âgées, est, pour une part très importante, lié à une perte d'acide hyaluronique.

Il a été constaté que les mono- ou poly-saccharides, constitutif d'une substance pectique provenant d'une plante de la famille des Lemnacées, selon l'invention, possèdent de nombreuses actions au niveau de la peau, notamment qu'elle permet de lutter contre les phénomènes du vieillissement cutané et qu'elle permet de protéger la peau contre tous types d'agressions extérieures. Ainsi, la composition selon l'invention peut être destinée à protéger les substrats kératiniques, et plus particulièrement à protéger la peau et/ou les phanères contre tous les types d'agressions extérieures.

L'utilisation de ces composés, ou d'une composition les contenant, va permettre aux substrats kératiniques d'être protégés et de mieux résister au stress que produit sur eux l'environnement. On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les frottements, l'eau à forte concentration de calcaire, les variations de température ou encore les produits à caractère irritant tels que les tensioactifs, les conservateurs ou les parfums.

Les mono- ou poly-saccharidcs, constitutifs d'une substance pectique provenant d'une plante de la famille des Lemnacées, ont donc une action efficace au niveau de la barrière cutanée. Ces composés augmentent donc la fonction de barrière cutanée de la peau, Ils favorisent ainsi la régénération tissulaire. Ainsi, les composés ou la composition selon l'invention pourront être utilisés afin d'augmenter la régénération tissulaire et/ou la cicatrisation de la peau.

Par ailleurs, l'invention concerne aussi l'utilisation, comme ingrédient actif, d'un mono-ou poly-sacchailde, constitutif d'une substance pectique provenant d'une plante de la famille des Lemnacées, pour la préparation d'une composition, les composés ou la composition étant destinés à traiter les manifestations de l'inflammation et de l'irritation cutanée. La composition est, en effet, très bien adaptée aux soins des peaux sensibles et fragiles.

Les composés ou la composition ont une action particulièrement efficace au niveau de la lutte contre les phénomènes allergiques, et notamment au niveau de ces conséquences sur la peau. Un autre aspect de l'invention, est donc l'utilisation des composés précités ou de la composition les contenant afin de lutter contre les phénomènes allergiques et/ou contre les manifestations de l'inflammation cutanée.

Par ailleurs, les composés selon l'invention, ont une action particulièrement efficace au niveau de la lutte contre les agents microbiens. Les composés ou la composition les contenant possèdent donc des propriétés anti-microbiennes, c'est-à-dire des propriétés anti-bactériennes, anti-tongiques, virocides ou encore anti-protozoaires. La composition aura ainsi une application particulièrement utile dans le traitement des infections cutanées, des phénomènes inflammatoires de la peau et/ou des cheveux ainsi que dans toutes les maladies de la peau et/ou des cheveux ayant une origine microbienne. Plus particulièrement, la composition selon l'invention aura une efficacité dans le traitement de la peau et dans la lutte contre les bactéries. Les composés selon l'invention auront ainsi nu effet bactériostatique.

Ainsi les composés ou la composition les contenant, selon l'invention, peuvent être utilisés dans le traitement des affections cutanées locales. De plus, ils permettront de lutter, d'une manière particulièrement efficace, contre l'acné et/ou contre toutes sortes d'infections ou de réactions inflammatoires.

Une autre particularité de la composition selon l'invention, est son action efficace dans la détoxification des cellules, c'est-à-dire que les composés ou la composition les contenant seront utiles pour détoxifier les cellules, et notamment les cellules de la peau.

Ces diverses propriétés peuvent être utilisées notamment pour réaliser des compositions destinées à protéger la peau contre les agressions extérieures provoquées, notamment, par l'action du rayonnement solaire ou par d'autres agents physiques, chimiques ou biologiques, ou encore des compositions permettant de lutter contre le vieillissement de la peau, permettant de l'entretenir et de la soigner.

Selon un autre aspect, la présente invention concerne un procédé de traitement cosmétique pour traiter les peaux, notamment les peaux âgées et/ou pour combattre les phénomènes de vieillissement cellulaire consistant à appliquer sur la surface de la peau une quantité efficace de la composition telle que définie précédemment, c'est-à-dire contenant au moins un mono- ou poly-saccharide, constitutif d'une substance pectique provenant d'une plante de la famille des Lemnacées, afin obtenir l'action désirée.

La présente invention concerne, de la même manière, un procédé de traitement cosmétique afin de protéger la peau et/ou les phanères contre tous types d'agressions extérieures, et/ou pour renforcer la fonction barrière de la peau et/ou des phanères.

Selon un autre aspect de l'invention, la présente invention concerne un procédé de traitement cosmétique afin de favoriser la cicatrisation et la régénération tissulaire, mais aussi afin de favoriser la différenciation cellulaire.

La présente invention concerne aussi, de la même manière, un procédé de traitement cosmétique afin de traiter les phénomènes allergiques et/ou inflammatoires de la peau, ainsi qu'un procédé afin de lutter contre les agents microbiens, qui consiste à appliquer sur la surface de la peau et/ou des phanères une quantité efficace de la composition telle que définie précédemment.

Des modes de réalisation particuliers de ce procédé de traitement cosmétique résultent également de la description précédente.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

### Exemple 1 - Préparation d'un extrait contenant des mono- ou poly-saccharides, constitutifs d'une substance pectique provenant d'une plante de la famille des Lemnacées.

Les lentilles d'eau, appartenant au genre Lemna, sont récoltées à la surface d'une étendue d'eau, puis lavées dans une eau très pure. 5 kg de ces lentilles fraîchement récoltées, ou conservées par congélation, sont broyées très finement, à l'aide d'un broyeur homogénéisateur de type ultra-turrax, dans 2 à 5 volumes d'une solution saline, préférentiellement dans une solution de chlorure de sodium. Cette opération est réalisée à une température comprise entre 4 et 90°C, préférentiellement à une température environ égale à 40°C.

Le broyat subit ensuite une étape de filtration et/ou de centrifugation ; le filtrat est éliminé. Cette opération d'extraction en solution saline, suivie d'une étape de centrifugation, est effectuée à trois reprises, à chaque fois les filtrats sont écartés. Le résidu est récupéré puis il est traité de la même manière, par extraction sous agitation puis par centrifugation et/ou filtration, trois fois, dans de l'eau. On obtient alors un broyat contenant les parois cellulaires des lentilles d'eau. Selon une variante du procédé d'extraction, cette étape peut être réalisée en présence d'enzymes, préférentiellement de type protéase et/ou cellulase.

L'extraction de la pectine, présente dans les parois cellulaires des lentilles d'eau, est réalisée en milieu aqueux, par une solution contenant un agent chélatant, l'oxalate d'ammonium, à une concentration comprise entre 0,02 et 0,1 M (l'EDTA ou le polyphosphate de sodium peuvent aussi être utilisés). Cette extraction est réalisée en milieu acide, à un pH compris entre 2 et 5 (avec de l'acide chlorhydrique), à une température comprise entre 4 et 90°C.

Les pectines ainsi extraites sont précipitées par des sels ou de l'alcool, elles sont ensuite recueillies et dialysées de manière à être purifiées. Les pectines ainsi obtenues peuvent être mises en solution aqueuse, constituant ainsi une première forme utilisable de l'extrait selon l'invention.

Les pectines ainsi obtenues peuvent être utilisées sous cette forme native ou bien sous une forme plus ou moins hydrolysée, c'est à dire sous une forme plus ou moins dépolymérisée. Dans ce cas, l'hydrolyse de la pectine peut être réalisée par différentes enzymes pectolitiques, cette hydrolyse peut également être effectuée de manière chimique, préférentiellement en milieu acide.

L'hydrolyse entraîne la formation de résidus de poids moléculaires plus ou moins faibles, on obtient ainsi des poly-saccharides, constitutifs d'une substance pectique, de différentes tailles.

Une hydrolyse totale libèrera tous les constituants de la pectine, et permettra d'obtenir un mélange, constitué, essentiellement, de mono-saccharides, contenant en majorité de l'acide galacturonique et de l'apiose.

Les différentes formes de pectines dépolymérisées, plus ou moins purifiées, constituent des formes d'extrait utilisables pour préparer la composition selon l'invention.

Selon un mode de réalisation particulier de l'invention, la pectine sera complètement hydrolysée de manière à obtenir des mono-saccharides constitutifs d'une substance pectique. Cette hydrolyse, suivie d'une étape de purification, permettra d'obtenir une solution contenant presque exclusivement de l'apiose.

Cette solution pourra constituer une forme d'extrait directement utilisable pour réaliser une composition cosmétique contenant de l'apiose.

### Exemple 2 - Préparation de compositions

Ces compositions ont été obtenues par simple mélange des différents composants. Les quantités indiquées sont données en pourcentage de poids.

### 1-mulsion huile dans eau

### Phase huileuse :

| | |
|---|---|
| ■ Montanov 68 (Cetearyl Alcohol and Cetearyl Glucoside) | 5,00 % |
| ■ Huile de Jojoba | 5,00 % |
| ■ Huile de Vaseline | 5,00 % |
| ■ Isopropyl Palmitate | 7,00 % |

### Phase aqueuse :

| | |
|---|---|
| ■ Glycérine | 5,00 % |
| ■ Allantoïne | 0,10 % |
| ■ Extrait de l'exemple 1 | 1,00 % |
| ■ Sepigel 305 (Polyacrylamide and C13-14 Isoparaffin and Laureth-7) | 0,30 % |
| ■ Conservateur | 0,50 % |
| ■ Parfum | 0,50 % |
| ■ Eau | qsp 100 % |

### 2-Gel

| | |
|---|---|
| ■ Carbopol Ultrez 10 (sol. à 2%) | 25,00 % |
| ■ Triéthanolamine | 0,50 % |
| ■ Extrait de l'exemple 1 | 5,00 % |
| ■ Conservateur | 0,20 % |
| ■ EDTA (séquestrant) | 0,10 % |
| ■ Parfum | 0,50 % |
| ■ Eau | qsp 100 % |

### 3 - Lotion

| | |
|---|---|
| ■ Mono Propylene Glycol | 1,00 % |
| ■ Allantoïne | 0,30 % |
| ■ Glycérine | 1,00 % |
| ■ Cetiol HE (PEG-7 Glyceryl Cocoate) | 1,00 % |
| ■ Extrait de l'exemple 1 | 0,50 % |
| ■ Conservateur | 0,20 % |
| ■ Parfum | 0,50 % |
| ■ Eau | qsp 100 % |

## Revendications

1. Utilisation d'au moins un mono- ou poly-saccharide, constitutif d'une substance pectique provenant d'une plante de la famille des Lemnacées, comme ingrédient actif, dans une composition cosmétique pour traiter, de manière curative et/ou préventive, les signes cutanés du vieillissement, et/ou améliorer l'aspect de la peau et/ou des phanères.

2. Utilisation d'au moins un mono- ou poly-saccharide, constitutif d'une substance pectique provenant d'une plante de la famille des Lemnacées, comme ingrédient actif, pour la préparation d'une composition pharmaceutique destinée à favoriser la régénération tissulaire et/ou la cicatrisation de la peau.

3. Utilisation d'au moins un mono- ou poly-saccharide, constitutif d'une substance pectique provenant d'une plante de la famille des Lemnacées, comme ingrédient actif, pour la préparation d'une composition pharmaceutique destinée à lutter contre les phénomènes allergiques cutanés.

4. Utilisation d'au moins un mono- ou poly-saccharide, constitutif d'une substance pectique provenant d'une plante de la famille des Lemnacées, pour la préparation d'une composition pharmaceutique destinée à lutter contre les manifestations de l'inflammation cutanée.

5. Utilisation d'au moins un mono- ou poly-saccharide, constitutif d'une substance pectique provenant d'une plante de la famille des Lemnacées, comme ingrédient actif, dans une composition cosmétique pour maintenir et/ou stimuler l'hydratation cutanée et/ou lutter contre le dessèchement de la peau.

6. Utilisation d'au moins un mono- ou poly-saccharide, constitutif d'une substance pectique provenant d'une plante de la famille des Lemnacées, comme ingrédient actif, pour la préparation d'une composition pharmaceutique destinée à détoxifier les cellules.

7. Utilisation d'au moins un mono- ou poly-saccharide, constitutif d'une substance pectique provenant d'une plante de la famille des Lemnacées, comme ingrédient actif, dans une composition pour protéger la peau et/ou les phanères contre les agressions extérieures, et/ou renforcer la barrière cutanée de la peau.

8. Utilisation d'au moins un mono- ou poly-saccharide, constitutif d'une substance pectique provenant d'une plante de la famille des Lemnacées, comme ingrédient actif, pour la préparation d'une composition cosmétique et/ou pharmaceutique destinée à une action anti-hyaluronidase.

9. Utilisation selon l'une des revendications 1 à 8, **caractérisée par le fait que** le poly-saccharide provenant de la famille des Lemnacées est une substance pectique comprenant au moins un motif apiose.

10. Utilisation selon la revendication 9, **caractérisée en ce que** le mono- ou poly-saccharide, constitutif d'une substance pectique provenant d'une plante de la famille des Lemnacées, est présent en une quantité représentant de 10-5 % à 20% du poids total de la composition, et préférentiellement en une quantité représentant de 10-4 % à 5% du poids total de la composition.

11. Utilisation selon l'une selon l'une des revendications 1 à 10, **caractérisée en ce que** la composition se présente sous la forme d'une composition adaptée à l'administration par voie topique cutanée, pour la peau, les muqueuses et/ou les phanères, comprenant, un milieu cosmétiquement ou pharmaceutiquement acceptable.

12. Utilisation selon l'une selon l'une des revendications 1 à 11, **caractérisée en ce que** les composés actifs sont préalablement solubilisés nu dispersés dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, la vaseline, une huile végétale ou tout mélange de ces solvants.

13. Utilisation selon l'une selon l'une des revendications 1 à 12, **caractérisée en ce que** les composés actifs sont préalablement solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

14. Utilisation selon l'une selon l'une des revendications 11 à 13, **caractérisée en ce que** la composition se présente sous forme d'une solution aqueuse, hydralcootique ou huileuse ou sous la forme d'une émulsion huile-dans-eau, eau-dans-huile ou émulsions multiples ou sous forme de crème, de suspensions, ou encore poudres ; ces compositions pouvant être plus ou moins fluides ou solides et avoir l'aspect d'une crème, d'une lotion, d'un lait, d'un sédum, d'une pommade, d'un gel, d'une pâte, d'une mousse ou d'un stick.

15. Procédé de traitement cosmétique pour traiter les peaux âgées et/ou pour combattre les phénomènes du vieillissement cellulaire, et/ou pour protéger la peau et/ou les phanères contre tous les types d'agressions extérieures, et/ou pour renforcer la barrière de la peau et/ou des phanères, consistant à appliquer sur la surface de la peau et/ou des phanères une quantité efficace d'une composition cosmétique et/ou dermatologique comprenant, comme principe actif, dans un milieu physiologiquement acceptable, une quantité efficace d'au moins un mono- ou poly-saccharide, constitutif d'une substance pectique provenant d'une plante de la famille des Lemnacées.

## Claims

1. Use of at least one mono- or poly-saccharide, consisting of a pectic substance obtained from a plant of the Lemnaceae family, as the active ingredient, in a cosmetic composition for treating, for curative and/or preventive purposes, the skin signs of ageing, and/or improving the appearance of the skin and/or skin appendages.

2. Use of at least one mono- or poly-saccharide, consisting of a pectic substance obtained from a plant of the Lemnaceae family, as the active ingredient, for preparing a pharmaceutical composition for promoting tissue regeneration and/or skin healing.

3. Use of at least one mono- or poly-saccharide, consisting of a pectic substance obtained from a plant of the Lemnaceae family, as the active ingredient, for preparing a pharmaceutical composition for combating skin allergy phenomena.

4. Use of at least one mono- or poly-saccharide, consisting of a pectic substance obtained from a plant of the Lemnaceae family for preparing a pharmaceutical composition for combating signs of skin inflammation.

5. Use of at least one mono- or poly-saccharide, consisting of a pectic substance obtained from a plant of the Lemnaceae family, as the active ingredient, in a cosmetic composition for maintaining and/or stimulating skin moisturisation and/or combating dry skin.

6. Use of at least one mono- or poly-saccharide, consisting of a pectic substance obtained from a plant of the Lemnaceae family, as the active ingredient, for preparing a pharmaceutical composition for detoxifying cells.

7. Use of at least one mono- or poly-saccharide, consisting of a pectic substance obtained from a plant of the Lemnaceae family, as the active ingredient, in a composition for protecting the skin and/or skin appendages against external damage and/or strengthening the skin barrier.

8. Use of at least one mono- or poly-saccharide, consisting of a pectic substance obtained from a plant of the Lemnaceae family, as the active ingredient, for preparing a cosmetic and/or pharmaceutical composition for an anti-hyalyuronidase action.

9. Use according to any of claims 1 to 8, **characterised in that** the polysaccharide from the Lemnaceae family is a pectic substance comprising at least one apiose structural unit.

10. Use according to claim 9, **characterised in that** the mono- or poly-saccharide, consisting of a pectic substance obtained from a plant of the Lemnaceae family is present in a quantity representing 10-5% to 20% of the total weight of the composition, and preferentially in a quantity representing from 10-4% to 5% of the total weight of the composition.

11. Use according to any of claims 1 to 10, **characterised in that** the composition is presented in the form of a composition suitable for administration by the cutaneous topical route, for the skin, mucosa and/or skin appendages, comprising a cosmetically or pharmaceutically acceptable medium.

12. Use according to any of claims 1 to 11, **characterised in that** the active compounds are previously solubilised or dispersed in one or a plurality of cosmetically or pharmaceutically acceptable solvents such as water, ethanol, propanol or isopropanol, propylene glycol, butylene glycol, dipropylene glycol, ethoxylated or propxylated diglycols, cyclic polyols, Vaseline, a vegetable oil or any mixture of said solvents.

13. Use according to any of claims 1 to 12, **characterised in that** the active compounds are previously solubilised in a cosmetic or pharmaceutical vector such as liposomes or adsorbed on powdered organic polymers, mineral substrates such as talc and bentonites, and more generally solubilised in, or fixed on, any cosmetically or pharmaceutically acceptable vector.

14. Use according to any of claims 11 to 13, **characterised in that** the composition is presented in the form of an aqueous, hydroalcoholic or oily solution or in the form of an oil-in-water, water-in-oil emulsion or multiple emulsions or in the form of cream, suspensions, or powders; these compositions optionally being more or less fluid or solid and having the appearance of a cream, lotion, milk, serum, ointment, gel, paste, foam or stick.

15. Cosmetic treatment method for treating aged skins and/or for combating cell ageing phenomena, and/or protecting the skin and/or skin appendages against all types of external damage, and/or strengthening the barrier of the skin and/or skin appendages, consisting of applying on the surface of the skin and/or skin appendages an effective quantity of a cosmetic and/or dermatological composition comprising, as the active ingredient, in a physiologically acceptable medium, an effective quantity of at least one mono- or polysaccharide, consisting of a pectic substance obtained from a plant of the Lemnaceae family.

## Patentansprüche

1. Verwendung von mindestens einem Mono- oder Polysacharid einer pektischen Substanz, stammend aus einer Pflanze der Familie der Wasserlinsengewächse, als Wirkstoff in einer kosmetischen Zusammensetzung, um auf heilende und/oder vorbeugende Weise die Alterungszeichen der Haut zu behandeln und/oder das Aussehen der Haut und/oder der Hautanhangsgebilde zu verbessern.

2. Verwendung von mindestens einem Mono- oder Polysacharid einer pektischen Substanz, stammend aus einer Pflanze der Familie der Wasserlinsengewächse, als Wirkstoff für die Zubereitung einer pharmazeutischen Zusammensetzung, um die Geweberegeneration und/oder die Narbenbildung der Haut zu begünstigen.

3. Verwendung von mindestens einem Mono- oder Polysacharid einer pektischen Substanz, stammend aus einer Pflanze der Familie der Wasserlinsengewächse, als Wirkstoff für die Zubereitung einer pharmazeutischen Zusammensetzung, um gegen die allergischen Phänomene der Haut anzukämpfen.

4. Verwendung von mindestens einem Mono- oder Polysacharid einer pektischen Substanz, stammend aus einer Pflanze der Familie der Wasserlinsengewächse, für die Zubereitung einer pharmazeutischen Zusammensetzung, um gegen die Erscheinungen der Hautentzündung anzukämpfen.

5. Verwendung von mindestens einem Mono- oder Polysacharid einer pektischen Substanz, stammend aus einer Pflanze der Familie der Wasserlinsengewächse, als Wirkstoff in einer kosmetischen Zusammensetzung, um die Hydratation der Haut aufrechtzuerhalten oder anzuregen und/oder gegen die Austrocknung der Haut anzukämpfen.

6. Verwendung von mindestens einem Mono- oder Polysacharid einer pektischen Substanz, stammend aus einer Pflanze der Familie der Wasserlinsengewächse, als Wirkstoff für die Zubereitung einer pharmazeutischen Zusammensetzung für die Entgiftung der Zellen.

7. Verwendung von mindestens einem Mono- oder Polysacharid einer pektischen Substanz, stammend aus einer Pflanze der Familie der Wasserlinsengewächse, als Wirkstoff in einer Zusammensetzung, um die Haut und/oder die Hautanhangsgebilde gegen äußere Aggressionen zu schützen und/oder die kutane Schranke der Haut zu verstärken.

8. Verwendung von mindestens einem Mono- oder Polysacharid einer pektischen Substanz, stammend aus einer Pflanze der Familie der Wasserlinsengewächse, als Wirkstoff für die Zubereitung einer kosmetischen und/oder pharmazeutischen Zusammensetzung für eine Antihyaluronidase-Wirkung.

9. Verwendung nach einem der Ansprüche 1 bis 8, **dadurch gekennzeichnet, dass** das Polysacharid, stammend aus der Familie der Wasserlinsengewächse, eine pektische Substanz ist, umfassend mindestens ein Apiosemotiv.

10. Verwendung nach Anspruch 9, **dadurch gekennzeichnet, dass** das Mono- oder Polysacharid einer pektischen Substanz, stammend aus einer Pflanze der Familie der Wasserlinsengewächse, in einer Menge vorhanden ist, die 10-5 % bis 20 % des Gesamtgewichts der Zusammensetzung darstellt, und vorzugsweise in einer Menge, die 10-4 % bis 5 % des Gesamtgewichts der Zusammensetzung darstellt.

11. Verwendung nach einem der Ansprüche 1 bis 10, **dadurch gekennzeichnet, dass** die Zusammensetzung die Form einer Zusammensetzung aufweist, die an die Verabreichung auf topisch kutanem Weg für die Haut, die Schleimhäute und/oder die Hautanhangsgebilde angepasst ist, umfassend ein kosmetisch oder pharmazeutisch akzeptables Medium.

12. Verwendung nach einem der Ansprüche 1 bis 11, **dadurch gekennzeichnet, dass** die Wirkstoffe zuvor in einem oder in mehreren kosmetisch oder pharmazeutisch akzeptablen Lösemitteln wie z.B. Wasser, Ethanol, Propanol oder Isopropanol, Propylenglykol, Butylenglycol, Dipropylenglykol, ethoxylierten oder propoxylierten Diglycolen, zyklischen Polyolen, Vaselin, pflanzlichem Öl oder allen Gemischen dieser Lösemittel solubilisiert oder dispegiert werden.

13. Verwendung nach einem der Ansprüche 1 bis 12, **dadurch gekennzeichnet, dass** die Wirkstoffe vorher in einem kosmetischen oder pharmazeutischen Vektor wie z.B. Liposomen solubilisiert oder auf pulverigen organischen Polymeren, mineralischen Trägern wie z.B. Talk und Bentoniten adsorbiert werden, und allgemeiner in allen kosmetisch oder pharmazeutisch akzeptablen Vektoren solubilisiert oder darauf fixiert werden.

14. Verwendung nach einem der Ansprüche 11 bis 13, **dadurch gekennzeichnet, dass** die Zusammensetzung die Form einer wässrigen, hydroalkoholischen oder öligen Lösung oder die Form einer Öl-in-Wasser-, Wasser-in-Öl-Emulsion oder von Mehrfachemulsionen oder die Form einer Creme, von Suspensionen oder auch Pulvern annimmt; wobei diese Zusammensetzungen mehr oder weniger flüssig oder fest sein und das Aussehen einer Creme, einer Lotion, einer Milch, eines Serums, einer Pomade, eines Gels, einer Paste, eines Schaums oder eines Sticks aufweisen können.

15. Verfahren der kosmetischen Behandlung, um gealterte Haut zu behandeln und/oder die Phänomene der Zellalterung zu bekämpfen und/oder die Haut und/oder die Hautanhangsgebilde gegen alle Arten von externen Aggressionen zu schützen und/oder um die Schranke der Haut und/oder der Hautanhangsgebilde zu verstärken, umfassend die Anbringung auf der Oberfläche der Haut und/oder der Hautanhangsgebilde einer wirksamen Menge einer kosmetischen und/oder dermatologischen Zusammensetzung, umfassend als Wirkstoff in einem physiologisch akzeptablen Medium eine wirksame Menge mindestens eines Mono- oder Polysacharids einer pektischen Substanz, stammend aus einer Pflanze der Familie der Wasserlinsengewächse.
